# EUROPEAN PATENT APPLICATION

(11) **EP 1 550 446 A1**
(43) Date of publication of application: **06.07.2005**
(21) Application number: 03026250.5
(22) Date of filing: 14.11.2003
(51) Int. Cl.: A61K 31/551, A61P 17/00

(54) **Use of 1-(5-isoquinolinesulfonyl) homopiperazine for treating and preventing hypopigmentary disorders**

(71) Applicant: Switch Biotech Aktiengesellschaft, 82061 Neuried (DE)
(72) Inventor: Bell, Stefan, 81379 München (DE); Ewald, Dagmar, 85402 Kranzberg (DE); Fritz, Stefanie, 81541 München (DE); Goppelt, Andreas, 80636 München (DE)
(74) Representative: Engelhard, Markus

(57) **Abstract**

The invention relates to the use of 1-(5-isoquinolinesulfonyl)homopiperazine, its isomers and salts for treating and/or preventing hypopigmentary disorders.

## Description

The invention relates to the use of a compound according to formula (I): 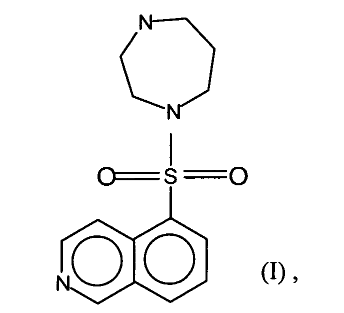 or of an isomer thereof, or of a pharmaceutically acceptable salt thereof.

In particular, the invention relates to the use of 1-(5-isoquinolinesulfonyl)homopiperazine , its isomers and pharmaceutically acceptable salts

The invention also relates to methods of treatment and or prevention of hypopigmentary disorders.

### State of the Art

In the skin or hair, melanocytes are the sole source of the pigment melanin. Melanin is synthesized within the melanocytes and later transferred to the surrounding keratinocytes. The colour of the skin is determined to a large extent by the amount and type of melanin within the epidermis. In general dysfunction of the melanocytes or the loss of the melanocytes itself leads to loss of pigmentation. The mechanisms for the destruction of melanocytes are likely to be multiple and complex, possibly a composite of several normal processes influencing melanocyte function, proliferation and/or survival. Also the pathomechanism of hyperpigmentary disorders is largely unclear.

Vitiligo, for example is a pigmentation disorder afflicting up to 2% of the worldwide population. It is a specific type of leukoderma manifested characteristically by depigmentation of the epidermis, best described as an acquired, progressive disorder that selectively destroys some or all melanocytes. The vitiligo disease is characterized by milky white macules on the skin, either due to missing melanin pigment or to complete absence of melanocytes in the dermo-epidermal junction of vitiligo areas. Vitiligo tends to be progressive throughout the life of affected individuals. Other disorders of hypopigmentation that are caused by a defect in melanin production or transfer include the Chediak-Higashi syndrome, Hermansky-Pudlak syndrome, the Waardenburg syndromes I-IV, the Angelman and Prader-Willi syndrome. Albinism instead is characterized by genetic defects that impede the synthesis of melanin. Piebaldism is characterized by the absence of melanin at birth due to a deficiency of melanocytes. During embryogenesis melanocytes fail to complete their migration from the neural crest to the epidermis. In vitiligo some melanocytes can be found in epidermis of early lesions that are only partially depigmented. In late lesions that were totally depigmented, there is complete absence of melanocytes. Also, there is a lack of knowledge about the pathophysiology and mechanisms underlying most pigmentary disorders.
At the moment there are still several hypotheses to explain the possible causes of vitiligo:
1) Autoimmune disease: Specific autoantibodies to melanocyte cell surface antigens are present in the circulation of most patients with vitiligo. These antibodies are unusual in persons with nonpigmentary skin diseases. Vitiligo antibodies are shown to have the functional capacity to kill pigment cells in vitro and can do so by two different mechanisms: complement-dependent cytotoxicity and antibody-dependent cellular cytotoxicity.
2) Self-destruction of melanocytes as a consequence of aberrant melanin biosynthesis: The autocytotoxic hypothesis is based on the observation that phenol and some of its derivatives are capable of killing pigment cells. Tyrosine, the substrate of the tyrosinase is itself a phenol derivative, is oxidized into melanin via a complex series of oxidative reactions. Some intermediates are capable of forming radicals. It is thought that melanin synthesis is confined within the melanosome to prevent these melanin precursors from diffusing into the cell where they might disrupt essential metabolic pathways.
3) Overproduction of neurotransmitters leading to death of melanocytes: Melanocytes are neural crest derived cells. A dysfunction of nervous function might be involved in the pathogenesis of vitiligo as shown by an altered balance of neuropeptides in vitiliginous skin. Neuropeptides are able to induce melanocyte dendricity and participate in the regulation of cell substrate adhesion, cell motility and shape. Neuropeptides may also regulate melanin synthesis or affect melanosomal transfer to surrounding keratinocytes.
4) More recently, also viral infections as well as oxidative stress and hormonal causes are discussed to be involved in the disease formation.
5) Although the pathogenesis of vitiligo is still not known, there is a genetic predisposition, demonstrated by the fact, that 40% of vitiligo patients have a positive family history for this disease.
6) A combination of some or all of above theories

Currently, no satisfying treatments exist for pigmentary disorders. For example, at present, there is no specific therapy for Vitiligo available without side effects and no innovative therapeutic programs are under development. No single therapy predictably produces good results in all patients and the responses are highly variable: systemic photochemotherapy (PUVA) gives satisfactory results only in some early disease states, however, treatment is time-consuming and has a high risk of developing cancer after prolonged treatment. Other therapies comprise systemic steroids,e.g. prednisone, hydrocortisone or triamcinolone, which however are also not suitable for prolonged treatment. In some cases, transplantation of skin has given positive results. In extreme cases, where the depigmented area has become very large, total chemical depigmentation of the skin is performed to achieve a homogeneous coloring of the skin.

Similarly, no specific and satisfactory treatment exists for Pityriasis alba, a common hypopigmented dermatitis that occurs primarily in school-aged children. Usually this disorder is left untreated as treatments with corticosteroids or retinoic acid or PUVA treatment are not very effecient.

Some pigmentary disorders, like vitiligo, have only skin manifestations limited to the pigmentation alterations. However, these disorders nevertheless pose severe psychological problems to the patients, as the sharp borders of depigmented areas are readily apparent to other persons, especially when occurring in the face. Vitiligo can be disfiguring and stigmatising, thereby causing significant psychological problems due to reduced social acceptance. Also, vitiligo usually persists for the whole life.

There is therefore a need for a specific treatment of hypopigmentary disorders, preferably vitiligo.

### Disclosure of the present invention

Surprisingly it was found that compounds according to formula (I): 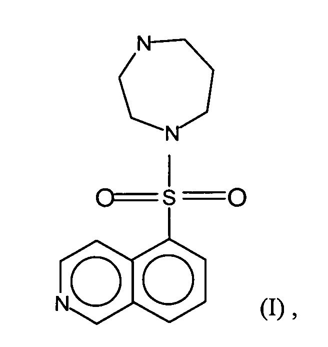 isomers thereof or pharmaceutically acceptable salts thereof, in particular 1-(5-isoquinolinesulfonyl)homopiperazine and its isomers and salts are useful in the treatment and prevention of hypopigmentary disorders, especially vitiligo.

Moreover it was surprisingly found that 1-(5-isoquinolinesulfonyl)homopiperazine is especially suitable for the treatment and prevention of hypopigmentary diseases which have an inflammatory and/or autoimmune component and/or in which T cell activation and proliferation plays a role, especially preferred vitiligo. Such inflammatory and/or autoimmune component may form part of the hypopigmentary disease or may be in addition thereto.

The present invention therefore relates to the use of a compound according to formula (I): 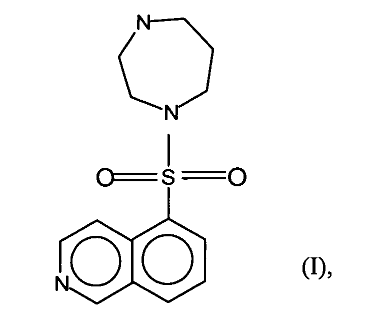 or an isomer thereof, or a pharmaceutically acceptable salt thereof, for the manufacture of a medicament for treating and/or preventing hypopigmentary disorders, especially vitiligo.

In a preferred embodiment, the compound is selected from the group consisting of:
1-(5-isoquinolinesulfonyl)homopiperazine, and pharmaceutically acceptable salts thereof, especially its hydrochloride salts.

In an even more preferred embodiment, the compound is selected from 1-(5-isoquinolinesulfonyl)homopiperazine, 1-(5-isoquinolinesulfonyl)homopiperazine hydrochloride anhydride, 1-(5-isoquinolinesulfonyl)homopiperazine hydrochloride hemihydrate and 1-(5-isoquinolinesulfonyl)homopiperazine hydrochloride trihydrate.

The invention also relates to a method of treating and/or preventing hypopigmentary disorders, especially vitiligo, comprising administering to a mammal a compound according to formula (I): 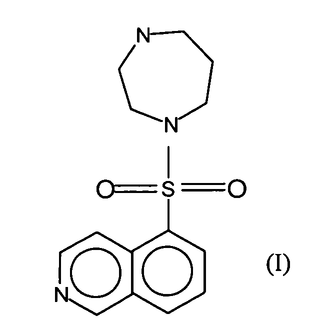 or an isomer thereof, or a pharmaceutically acceptable salt thereof.

In a preferred embodiment, the compound is selected from the group consisting of:
1-(5-isoquinolinesulfonyl)homopiperazine, and pharmaceutically acceptable salts thereof, especially its hydrochloride salts.

In an even more preferred embodiment, the compound is selected from 1-(5-isoquinolinesulfonyl)homopiperazine, 1-(5-isoquinolinesulfonyl)homopiperazine hydrochloride anhydride, 1-(5-isoquinolinesulfonyl)homopiperazine hydrochloride hemihydrate and 1-(5-isoquinolinesulfonyl)homopiperazine hydrochloride trihydrate.

In one embodiment, the hypopigmentary disorder has an inflammatory and/or an autoimmune component. Preferably the hypopigmentary disorder is selected from albinism, vitiligo, postinflammatory hypopigmentation, piebaldism, Pityariasis alba, Hypomelanoses, Leukodermas, hypopigmentation occurring e.g. after externally induced peels, e.g. chemical peels, e. g. with phenol, or laser or cryo-surgery of the skin, Chediak-Higashi syndrome, Hermansky-Pudlak syndrome, the Angelman and Prader-Willi syndrome, wherein, more preferably, the hypopigmentary disorder is a disorder in which T cell activation and proliferation plays a role, and which hypopigmentary disorder is more preferably selected from post-inflammatory hypopigmentation and vitiligo. In the most preferred embodiment, the hypopigmentary disorder is vitiligo.

1-(5-isoquinolinesulfonyl)homopiperazine, also called Fasudil is a compound with vasodilatory actions which is currently in clinical trials for treating cerebral vasospasms (EP 0 870 767, EP 0 187 371B1). US 6,271,224 discloses the use of Fasudil for the treatment of glaucoma and ocular ischemia. Also, Fasudil has been proposed for infectious disease (JP10045598A2), mental disorder syndrome (JP06293643A2), controlling cancer invasion and the effect of controlling carcinomatous peritonitis (EP 1064944).

Methods of production of the compounds 1-(5-isoquinolinesulfonyl)homopiperazine or 1-(5-isoquinolinesulfonyl)homopiperazine hydrochloride in anhydrous form and as hydrates according to the present invention are well known to someone skilled in the art, and are described e.g. in EP 0 870 767B1, the contents of which is hereby incorporated by reference.

### Hypopigmentary disorders

Hypopigmentary disorders according to the present invention are non-malignant disorders of the skin in mammals, including humans which are characterized by a decrease in pigmentation of the skin compared to healthy individuals. Such decrease in pigmentation may occur locally, as e.g. in mild forms of vitiligo, or may affect the whole skin. Such decrease in pigmentation may result in total loss of pigmentation, as e.g. in affected skin areas of vitiligo patients, or may result in "lighter" but still pigmented skin as in Pityriasis alba. In some embodiments, the hypopigmentary disorders, according to the present invention, have an inflammatory and/or an autoimmune component. As used herein, the term "having an inflammatory component" is meant to designate any condition which is accompanied locally or temporally with syndromes characteristic of an inflammatory reaction, such as the induction of certain cytokines, in particular soluble IL-2R (sIL-2R), IL-6 and IL-8, and increased levels of inflammatory cells, in particular T-cells and macrophages, at sites of lesions or around lesions. Such inflammatory and/or an autoimmune component may form part of the hypopigmentary disorder or may be in addition thereto.

The term "accompanied temporally" may mean that the inflammatory component precedes the hypopigmentary disorder, is concomitant therewith or follows it.

The term "autoimmune component" in an organism is meant to designate any condition which is characterized by a reaction of the organism's own immune system against the organism itself or tissues or cells or other components thereof. An example of such a reaction is the production of auto-antibodies which are antibodies that are directed at some of an organism's own tissues or cells or other body components.

Examples of hypopigmentary disorders are, but not limited to, albinism, vitiligo, postinflammatory hypopigmentation, piebaldism, Pityariasis alba, Hypomelanoses, Leukodermas, hypopigmentation occuring e.g. after externally induced peels, e.g. chemical peels with phenol, or laser or cryo-surgery of the skin, Chediak-Higashi syndrome, Hermansky-Pudlak syndrome, the Angelman and Prader-Willi syndrome. An especially preferred hypopigmentary disorder of the present invention is vitiligo.

Examples of hypopigmentary disorders in which T cell activation and proliferation plays a role are postinflammatory hypopigmentation and vitiligo, preferably vitiligo.

Treatment according to the present invention relates to the complete or partial healing of the hypopigmentary disorder in mammals, including humans as well as to the stop or slowing-down of the progression of a hypopigmentary disorder. Also, the compounds of the present invention are suitable for the prevention of a hypopigmentary disorder in mammals, including humans.

Treatment of vitiligo according to the present invention relates to the complete or partial healing of the disorder vitiligo; i.e. complete or partial repigmentation of existing white macules on vitiligo patients, as well as to the stop or slowing-down of the extension of the white macule areas on vitiligo patients. Prevention of vitiligo according to the present invention relates to the prevention of occurrence of vitiligo phenotype in to date unaffected persons. Preferably, such unaffected persons are persons with higher risk of vitiligo; i.e. persons with a family history of vitiligo.

### Pharmaceutical compositions

### Pharmaceutically acceptable salts

Examples of pharmaceutically acceptable addition salts include, without limitation, the nontoxic inorganic and organic acid addition salts such as the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enantate derived from enanthic acid, the formate derived from formic acid, the fumarate derived from fumaric acid, the glutamate derived from glutamic acid, the glycolate derived from glycolic acid, the hydrochloride derived from hydrochloric acid, the hydrobromide derived from hydrobromic acid, the lactate derived from lactic acid, the maleate derived from maleic acid, the malonate derived from malonic acid, the mandelate derived from mandelic acid, the methanesulfonate derived from methane sulphonic acid, the naphthalene-2-sulphonate derived from naphtalene-2-sulphonic acid, the nitrate derived from nitric acid, the perchlorate derived from perchloric acid, the phosphate derived from phosphoric acid, the phthalate derived from phthalic acid, the salicylate derived from salicylic acid, the sorbate derived from sorbic acid, the stearate derived from stearic acid, the succinate derived from succinic acid, the sulphate derived from sulphuric acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

An especially preferred salt according to the present invention is the hydrochloride, especially the dihydrochloride.

In another embodiment, the 1-(5-isoquinolinesulfonyl)homopiperazine is used in its free base form according to the present invention.

Metal salts of a chemical compound of the invention includes alkali metal salts, such as the sodium salt of a chemical compound of the invention containing a carboxy group.

The chemical compounds of the invention may be provided in unsolvated or solvated forms together with a pharmaceutically acceptable solvents such as water, ethanol, and the like. Solvated forms may also include hydrated forms such as the monohydrate, the dihydrate, the hemihydrate, the trihydrate, the tetrahydrate, and the like. In general, solvated forms are considered equivalent to unsolvated forms for the purposes of this invention. However, in case of 1-(5-isoquinolinesulfonyl)homopiperazine hydrochloride, some hydrates have proven to be specifically suitable for tablettation, namely the hemihydrate and trihydrate (EP 0870767B1). Such hydrates are preferred hydrates of 1-(5-isoquinolinesulfonyl)homopiperazine hydrochloride and dihydrochloride useable according to the present invention.

### Administration and Formulation

The production of medicaments containing 1-(5-isoquinolinesulfonyl)homopiperazine, its isomers and salts according to the invention and their application can be performed according to well-known pharmaceutical methods.

While 1-(5-isoquinolinesulfonyl)homopiperazine useable according to the invention for use in therapy may be administered in the form of the raw chemical compound, it is preferred to introduce the active ingredient, optionally in the form of a physiologically acceptable salt, especially preferred in form of its hydrochloride salt, in a pharmaceutical composition together with one or more adjuvants, excipients, carriers, buffers, diluents, and/or other customary pharmaceutical auxiliaries. Such salts of 1-(5-isoquinolinesulfonyl)homopiperazine may be anhydrous or solvated, in case of the preferred hydrochloride salt, the salt preferably may be anhydrous, or solvated, especially in form of its hemidrate or trihydrate.

In a preferred embodiment, the invention provides medicaments comprising a compound useable according to the invention, or a pharmaceutically acceptable salt or derivative thereof, together with one or more pharmaceutically acceptable carriers therefor, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules. In case of oral administration by tablets, the use of 1-(5-isoquinolinesulfonyl)homopiperazine hydrochloride hemihydrate or trihydrate is especially preferred due to their good shapeability and tablettability (EP 0870767B1). An oral sustained release formulation for compounds useable according to the invention is described in EP 1 110 553.

The compounds useable according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such Medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The compound useable according to the invention can be administered in a wide variety of oral and parenteral dosage forms. It will be obvious to those skilled in the art that the following dosage forms may comprise, as the active component, either a compounds useable according to the invention or a pharmaceutically acceptable salt of a compounds useable according to the invention.

For preparing a medicament from a compound useable according to the invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify., Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.
Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

In an especially preferred embodiment of the present invention the medicament is applied topically. This reduces possible side effects and limits the necessary treatment to those areas affected. For topical administration, the use of 1-(5-isoquinolinesulfonyl)homopiperazine, and its hydrochloride form are preferred, although also other salts and isomers may be used.

Preferably the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste oder a powder.

Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.).

### Assays for testing subject compounds

In general, tests for measuring the effect on hypopigmentary disorders are described in the prior art. For example, an in vitro model consisting of keratinocytes and melanocytes may be used. E.g. the commercially available MelanoDerm Skin Model (MatTek Corporation, Ashland, Massachusetts) uses NHEK (normal human derived keratinocytes)-NHEM (normal human derived melanocytes) cocultures (MelanoDerm Skin model) to model the human epidermis taking into account that melanocytes are dependent on keratinocyte signalling. NHEKs are cultivated in culture inserts placed on the NHEM monolayers according to the instructions of the manufacturer (MatTek Corporation, Ashland, Massachusetts). NHEMs undergo spontaneously melanogenesis up to 3 weeks of culturing. Regarding evaluation of pharmaceutical agents to stimulate skin pigmentation as needed for treating hypopigmentary disorders, the tissues darken faster than untreated controls.

Other in vitro assays for determining the effect of a subject compound on hypopigmentary disorders are in vitro assays based on melanin content analysis.

Melanin content analysis assays are also described in the prior art.

For example, treated and control melanocytes are harvested by trypsinization. Ten percent of the melanocytes are removed and counted on a Coulter counter. The remaining melanocytes are centrifuged and the resulting cell pellet is washed in phosphate-buffered saline pH 7.4 and recentrifuged. The cell pellet is then dissolved in 1ml of 1 M sodium hydroxide. The optical density of this solution is measured spectrophotometrically at 405 nm and is compared to synthetic melanin to determine melanin content per cell. A higher melanin content reflects enhanced pigmentation.

Similarly, the assay can be performed as follows: treated and control melanocytes are harvested by trypsinization, washed with phosphate-buffered saline (PBS), suspended in 500 µl PBS, and counted. The cells are then solubilized by adding 500 µl of 10N NaOH and incubating for 30 min at room temperature. Absorbance at 475 nm is measured and compared with a standard curve for synthetic melanin.

Alternatively, the ¹⁴C-tyrosine melanin-formation assay can be used, which measures the radioactive melanin formed when ¹⁴C-tyrosine is converted to the acid-insoluble melanin biopolymer. For this assay, cell extracts from treated and control cells are incubated with ¹⁴C-tyrosine for 1 hour and absorbed on filter paper. The filter paper is dried then washed in 0.1 mol/L hydrochloric acid three times and placed in a scintillation vial with 5 ml scintillator solution. The radioactivity is counted in triplicate using the same scintillator counter.

Alternatively, the effect of a compound on a hypopigmentary disorder can be determined by measuring tyrosinase activity, which are well described in the prior art.

For example, tyrosinase activity of cell culture can be determined: treated and control melanocytes are incubated with 1.0 µCi [³H]tyrosine per ml. 700 µl of 10% trichloroacetic acid containing 20% charcoal (charcoal solution) is added to the medium (700 µl), and the mixture is mixed in a vortex for 30 s and then centrifuged at 10,000 rpm for 10 min. Seven hundred microliters of the supernatant is transferred into a new tube and treated twice with the charcoal solution. The radioactivity of the final supernatant is determined in a liquid scintillation counter.

Alternatively, tyrosinase activity can be determined in cell extracts. For example, treated and control melanocytes are collected and washed twice with PBS, and then lyzed in 0.1 M sodium phosphate buffer (pH 6.8) containing 1% Triton X-100. After determining the protein content in the cell extract, 10 µg of each extract is incubated in 100 µl of 100 mM sodium phosphate buffer (pH 6.8) containing 1.0 µCi [³H]tyrosine per ml, 5 µg L-dihydroxyphenylalanine, and 1% Triton X-100 for 15 min at 37°C. After adding 900 µl of charcoal solution, the samples stand for 20 min at 4°C and then are centrifuged at 10,000 rpm for 10 min. The supernatants are applied to a 0.2 ml Dowex-50 column equilibrated in 10% trichloroacetic acid and washed with 0.5 ml of 10% trichloroacetic acid, after which the radioactivity of the effluents is determined in the liquid scintillation counter.

Also, some animal models of varying predictability and suitability exist. An evaluation of suitability of various vitiligo animal models is described in Vitiligo (eds. S. Hann and J.

J.Nordlund, Blackwell Science, Chapter 33: Animal models by L. Lamoreux and R. E. Boissy)

For example, the Smyth chicken exhibiting a genetically inherited form of vitiligo-like depigmentation resulting from the loss of melanocytes in feathers is discussed as vitiligo model. The pigmentation phenotype of this animal model appears to involve an immune response. Also, the so-called vitiligo mouse is described. In this model no immune component is involved and the disorder is not polyfactorial; i.e. unlike in humans.

Vitiligo, for example, is not a simple, one-locus-disease, and inheritance is not its only cause. Therefore, in the case of vitiligo, animal models must be used with the knowledge that the disorder observed in the genetically defective animal will not be homologous with the disorder of the average human vitiligo patient. Thus, the study of one animal model, especially if the defect is caused by one gene locus in the model, need not be more predictive than an in vitro model.

None of the aforementioned tests has proved particular useful on its own when it came to identifying compounds useful in the treatment and/or prevention of hypopigmentary disorders, given that not many compounds are known to have a pronounced positive effect on the onset of hypopigmentary disorders,. This shortcoming of the prior art has now been overcome, in that the present inventors have found that surprisingly 1-(5-isoquinolinesulfonyl)homopiperazine and its isomers and salts useable according to the present invention, as defined before, provide for compounds which have a beneficial effect on hypopigmentary disorders, especially on vitiligo. Therefore, according to another aspect of the present invention, it provides for the use of 1-(5-isoquinolinesulfonyl)homopiperazine and its isomers and salts , as defined above, in a screening assay for compounds which are suitable for the treatment and/or prevention of hypopigmentary disorders. In one embodiment, such screening assay is an in-vitro-model consisting of a co-culture of keratinocytes and melanocytes as described above. In another embodiment the screening assay is an in-vitro-assay based on melanin content analysis, as described above. In another embodiment, the screening assay is based on the measurement of tyrosinase activity, as described above. In yet another embodiment, the screening assay is based on an animal model, as described above.

In all these screening assays the 1-(5-isoquinolinesulfonyl)homopiperazine and/or its isomers and/or salts useable according to the present invention serve as positive controls in that they inhibit/prevent or induce the onset of hypopigmentary disorders,especially vitiligo and the response of the assay system towards these controls is compared with the response of the assay system towards a subject compound to be tested.

Further aspects of the present invention will become evident by the figures and the following examples which are given to merely illustrate the invention, not to limit the same.

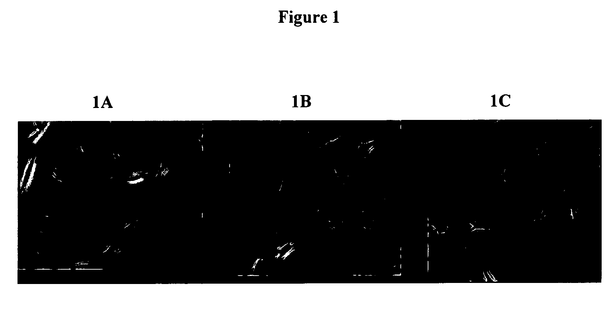

Figure 1 shows the influence of 1-(5-isoquinolinesulfonyl)homopiperazine on dendrite formation in melanocytes in a melanocyte dendrite outgrowth assay described below. Image 1A shows control melanocytes in buffer, showing no dendrites. Image 1B shows melanocytes incubated with Forskolin as positive control, resulting in drendrite formation. Image 1C shows melanocytes incubated with 1-(5-isoquinolinesulfonyl)homopiperazine, resulting in strong dendrite formation.

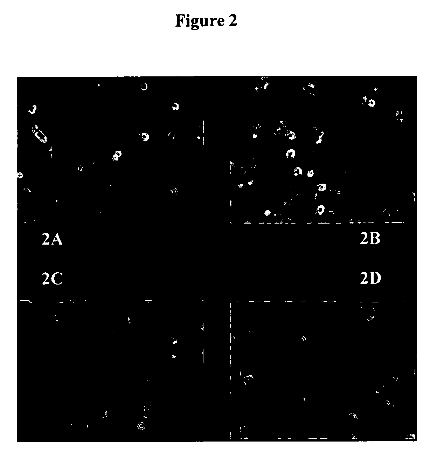

Figure 2 shows the influence of 1-(5-isoquinolinesulfonyl)homopiperazine on keratinocyte morphology as a measure for cytotoxicity. Figures 2A and 2C show control HaCaT and NHEK keratinocyte cells incubated in buffer with normal morphology. Figures 2B and 2D show HaCaT and NHEK keratinocyte cells treated with 1-(5-isoquinolinesulfonyl)homopiperazine. These treated cells also showed normal morphology, proving that of 1-(5-isoquinolinesulfonyl)homopiperazine is not cytotoxic for keratinocytes.

### Example 1: Effect of 1-(5-isoquinolinesulfonyl)homopiperazine on melanocyte function

A possibility to test the effect of a compound on melanocyte function as a measure for pigmentation, the melanocyte dendrite outgrowth assay can be performed. Vitiligo melanocytes for example have only stubby dendrites. Increasing dendricity as a prerequisite for the transfer of the melanosomes from melanocytes to the surrounding keratinocytes rescues this defect.

For this assay, cells from a human melanoma cell line (Mel-Ho) were obtained from DSMZ (Deutsche Sammlung für Mikroorganismen und Zellkulturen) and were cultured according to the protocol provided by DSMZ. In short, after thawing the melanocytes were cultured in T-75 flasks in melanocyte culture media (provided by the manufacturer) until they reach 70-80% confluency. The melanocytes were then trypsinized, transferred into 5-10 new T-75 flasks, and grown again to same confluency as described above. After this expansion step the melanocytes were immediately used for the Melanocyte Dendrite Outgrowth Assay or were frozen for later use.

For the assay, the melanocytes were seeded in 24 well plates at a density of 1-5 x104 cells/well. After 24 hours, the melanocytes were treated with 20µM Forskolin (Sigma-Aldrich) or different concentrations of 1-(5-isoquinolinesulfonyl)homopiperazine dihydrochloride (Sigma-Aldrich) for 24 hours. Forskolin is known to cause increased melanocyte dendricity and was used as a positive control. Cells treated with media only were used as a negative control. Melanocyte dendrite outgrowth was documented by digital photography (Canon G2 Powershot) after 3, 6 and 24 hours. A representative result is shown in Figure 1. Image A of Figure 1 shows the negative control with treated with media only, exhibiting no dendrites, whereas in the positive control, i.e. Forskolin-treated cells, dendrites are clearly visible. Surprisingly it was found that also with 1-(5-isoquinolinesulfonyl)homopiperazine dihydrochloride-treated cells, dendricity was strongly stimulated (Image 3 of Figure 1). In this experiment 100 µM were used, however also lower concentrations of 1-(5-isoquinolinesulfonyl)homopiperazine dihydrochloride are sufficient to induce dendrite formation.

In summary, the strongly increased dendricity induced by 1-(5-isoquinolinesulfonyl)homopiperazine dihydrochloride proves good suitability of the compounds useable according to the invention for treating and/or preventing hypopigmentary disorders, especially vitiligo.

### Example 2: Effect of 1-(5-isoquinolinesulfonyl)homopiperazine on T cell activation

As mentioned above, vitiligo is a hypopigmentary disorder with an unclear pathomechanism and various hypotheses on the causes of vitiligo exist. Possibly, several mechanisms contribute to the onset and/or progression of the disorders. It would be advantageous, if a compound useable according to the invention additionally acted on another discussed pathomechanism of vitiligo. It was therefore investigated, whether 1-(5-isoquinolinesulfonyl)homopiperazine has an inhibitory effect on T cell activation. An inhibitory action on T cell activiation would prove that 1-(5-isoquinolinesulfonyl)homopiperazine is not only beneficial for supporting the dysfunctional melanocytes for regaining their functionality by increasing dendricity but has in addition an inhibitory action on T cells which, according to the autoimmune thesis, play a role in the destruction of the melanocytes.

T cell activation was determined by measuring cytokine secretion and BrDU incorporation, i.e. by methods well known to a person skilled in the art. In short, peripheral blood mononuclear cells (PBMCs) were isolated from peripheral blood and were activated with soluble anti-CD3-antibody. The inhibitory action of 1-(5-isoquinolinesulfonyl)homopiperazine on T cell activation and proliferation was determined by measuring IL-2 in the supernatant by means of ELISA (T cell activation) and by the incorporation of BrDU (T cell proliferation). It was found that 1-(5-isoquinolinesulfonyl)homopiperazine dihydrochloride inhibits T cell activation and proliferation with an IC₅₀ of 90 µM.

Surprisingly it was found that 1-(5-isoquinolinesulfonyl)homopiperazine has an inhibitory activity on T cell activation, which makes the compounds useable according to the invention especially suitable for treating and/or preventing hypopigmentary diseases in which T cell activation and proliferation plays a role, especially preferred vitiligo.

### Example 3: Effect of 1-(5-isoquinolinesulfonyl)homopiperazine on keratinocytes

1-(5-isoquinolinesulfonyl)homopiperazine or its salts and isomers have never been tested or considered for topical application before, and hence, no data exist on the suitability of the 1-(5-isoquinolinesulfonyl)homopiperazine and its salts and isomers for topical application; notably, it is not known whether the substance exhibits cytotoxic effects on keratinocytes, which on the one hand represent by far the predominant cell type of the skin and on the other hand is the cell type which obtains melanin by the melanocytes by transfer via the dendrites of the melanocytes. Any adverse effect of 1-(5-isoquinolinesulfonyl)homopiperazine on keratinocytes could therefore even worsen the hypopigmentary disorder and/or could lead to other skin disorders, like eczema, dermatoses or erythemas. Therefore cytotoxicity of 1-(5-isoquinolinesulfonyl)homopiperazine on keratinocytes was tested.

To test a possible cytotoxic effect resulting in a morphological change of keratinocytes, normal human epidermal keratinocytes were obtained from Clonetics (neonatal keratinocytes, NHEK) and were cultured according to the manufacturers' protocol. In short, after thawing, the keratinocytes were cultured in T-75 flasks in keratinocyte culture media (provided by the manufacturer) until they reached 70-80% confluency. The keratinocytes were then trypsinized, transferred into new T-75 flasks at a density of 3500 cells/cm². After this expansion step the keratinocytes were immediately used for the cytotoxic assay procedure or were frozen for later use. Normal human epidermal keratinocytes or HaCaT keratinocytes cells were seeded in 24 well plates at a density of 1-5 x104 cells/well. After 24 hours, the keratinocytes were treated with different concentrations of 1-(5-isoquinolinesulfonyl)homopiperazine (Sigma-Aldrich) for 24 hours. Cells treated with media only were used as a negative control. Photos were taken at 3, 6 and 24 hours after stimulation for documentary reasons. A representative result is shown in Figure 2. Image 2B shows HaCaT keratinocytes treated with 1-(5-isoquinolinesulfonyl)homopiperazine dihydrochloride, whereas Image 2A shows control HaCaT cells incubated in buffer only. No morphological changes were observed in cells treated with 1-(5-isoquinolinesulfonyl)homopiperazine dihydrochloride, proving that the substance has no cytotoxic effect on the cells. Image 2D shows NHEK keratinocytes treated with 1-(5-isoquinolinesulfonyl)homopiperazine dihydrochloride, whereas Image 2C shows control NHEK cells incubated in buffer only. No morphological changes were observed in cells treated with 1-(5-isoquinolinesulfonyl)homopiperazine dihydrochloride, proving that the substance has no cytotoxic effect on the NHEK cells. In summary, no morphological change after 24h stimulation were detected in any of the two keratinocyte cell lines, proving that 1-(5-isoquinolinesulfonyl)homopiperazine has no cytotoxic effects on keratinocytes and is therefore suitable for topical administration of the drug, which is the preferred route of administration for treating hypopigmentary disorders, especially vitiligo.

### Example 4: Effect of 1-(5-isoquinolinesulfonyl)homopiperazine on vitiligo patients

In order to prove suitability of 1-(5-isoquinolinesulfonyl)homopiperazine for the treatment of vitiligo, 25 to 30 vitiligo vulgaris patients are treated once or twice daily with a topical formulation of 1-(5-isoquinolinesulfonyl)homopiperazine (0.1%-10% preferably 0.1%-10% by weight of 1-(5-isoquinolinesulfonyl)homopiperazine) for 3 to 6 months. Already existing macules are treated with the medicament. The surface area of treated lesions (i.e. white macules) is documented regularly by photography. It is expected that 1-(5-isoquinolinesulfonyl)homopiperazine is efficient in preventing further spreading of vitiligo and/or inducing repigmentation in treated macules.

The features of the present invention disclosed in the specification, the claims and/or in the accompanying drawings, may, both separately, and in any combination thereof, be material for realising the invention in various forms thereof. All citations included herein are incorporated herein by reference.

## Claims

1. Use of a compound according to formula (I) 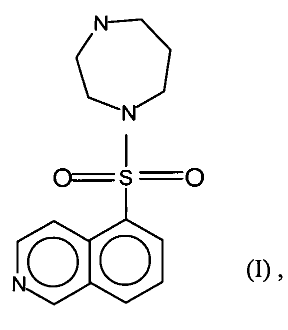 or an isomer thereof, or a pharmaceutically acceptable salt thereof, for the manufacture of a medicament for the treatment and/or prevention of hypopigmentary disorders, especially vitiligo.

2. Use according to claim 1, **characterized in that** the hypopigmentary disorder has an inflammatory and/or an autoimmune component.

3. Use according to claim 1 - 2, **characterized in that** the compound is selected from 1-(5-isoquinolinesulfonyl)homopiperazine, and pharmaceutically acceptable salts thereof, especially its hydrochloride salts.

4. Use according to any of claims 1 to 3, **characterized in that** the hypopigmentary disorder is selected from albinism, vitiligo, postinflammatory hypopigmentation, piebaldism, Pityariasis alba, Hypomelanoses, Leukodermas, hypopigmentation occurring e.g. after externally induced peels, e.g. chemical peels, e. g. with phenol, or laser or cryo-surgery of the skin, Chediak-Higashi syndrome, Hermansky-Pudlak syndrome, the Angelman and Prader-Willi syndrome.

5. Use according to any of claims 1 to 4, **characterized in that** the hypopigmentary disorder is a disorder in which T cell activation and proliferation plays a role, preferably selected from post-inflammatory hypopigmentation and vitiligo.

6. Use according to any of claims 1 to 5, **characterized in that** the hypopigmentary disorder is vitiligo.

7. Use according to any of claims 1 to 6, **characterized in that** the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste or a powder, or a solution or suspension.

8. Use according to any of claims 1 to 7, **characterized in that** the compound is applied topically or systemically or via a combination of the two routes, preferably topically.
